# NEW EUROPEAN PATENT SPECIFICATION

(11) **EP 2 268 804 B2**
(45) Date of publication and mention of the opposition decision: **09.08.2023**
(45) Mention of the grant of the patent: 13.09.2017
(21) Application number: 09721293.0
(22) Date of filing: 20.03.2009
(51) Int. Cl.: C12N 9/24

(54) **HEMICELLULASE ENRICHED COMPOSITIONS FOR ENHANCING HYDROLYSIS OF BIOMASS**
MIT HEMICELLULASEN ANGEREICHERTE ZUSAMMENSETZUNGEN ZUR ERHÖHUNG DER HYDROLYSE
COMPOSITIONS ENRICHIES EN HÉMICELLULASE POUR AMÉLIORER L'HYDROLYSE DES BIOMASSES

(30) Priority: 21.03.2008 US 38520
(43) Date of publication of application: 05.01.2011
(73) Proprietor: Danisco US Inc., Palo Alto, CA 94304 (US)
(72) Inventor: POWER, Scott D., DANISCO US INC.,GENENCOR DIVISION, Palo Alto, CA 94304 (US); CALDWELL, Robert M., DANISCO US INC.,GENENCOR DIVISION, Palo Alto, CA 94304 (US); LANTZ, Suzanne E., DANISCO US INC.,GENENCOR DIVISION, Palo Alto, CA 94304 (US); LARENAS, Edmund, A., DANISCO US INC.,GENENCOR DIVISION, Palo Alto, CA 94304 (US)
(74) Representative: Mewburn Ellis LLP
(86) International application number: PCT/US2009/037853
(87) International publication number: WO 2009/117689

(56) References cited:
- WO-A-03/093420
- WO-A-2006/114095
- DE-A1- 10 043 662
- US-A1- 2002 084 046
- SORENSEN H R ET AL: "Enzymatic hydrolysis of water-soluble wheat arabinoxylan. 1. Synergy between alpha-L-arabinofuranosidases, endo-1,4-beta-xylanases, and beta-xylosidase activities" BIOTECHNOLOGY AND BIOENGINEERING, WILEY & SONS, HOBOKEN, NJ, US, vol. 81, no. 6, 20 March 2003 (2003-03-20), pages 726-731, XP002386252 ISSN: 0006-3592
- THYGESEN ANDERS ET AL: "Production of cellulose and hemicellulose-degrading enzymes by filamentous fungi cultivated on wet-oxidised wheat straw" ENZYME AND MICROBIAL TECHNOLOGY, STONEHAM, MA, US, vol. 32, no. 5, 8 April 2003 (2003-04-08), pages 606-615, XP002412141 ISSN: 0141-0229
- SHALLOM DALIA ET AL: "Microbial hemicellulases." CURRENT OPINION IN MICROBIOLOGY, vol. 6, no. 3, June 2003 (2003-06), pages 219-228, XP002536339 ISSN: 1369-5274
- TORRONEN A ET AL: "THE TWO MAJOR XYLANASES FROM TRICHODERMA REESEI: CHARACTERIZATION OF BOTH ENZYEMES AND GENS" BIO/TECHNOLOGY, NATURE PUBLISHING CO. NEW YORK, US, vol. 10, 1 November 1992 (1992-11-01), pages 1461-1465, XP001056543 ISSN: 0733-222X
- OGASAWARA W ET AL: "Cloning, functional expression and promoter analysis of xylanase III gene from Trichoderma reesei" APPLIED MICROBIOLOGY AND BIOTECHNOLOGY, SPRINGER, BERLIN, DE, vol. 72, no. 5, 7 March 2006 (2006-03-07), pages 995-1003, XP019441655 ISSN: 1432-0614
- FOREMAN et al.: "Transcriptional Regulation of Biomass-degrading Enzymes in the Filamentous Fungus Trichoderma reesei", The Journal of Biological Chemistry, vol. 278, 2003, pages 31988-31997,

## Description

### PRIORITY

The present application claim priority to U.S. Provision Application Serial No. 61/038,520, filed on March 21, 2008.

### TECHNICAL FIELD

The present compositions and methods relate to cellulase/hemicellulase enzyme blends for improving the enzymatic hydrolysis of cellulosic materials.

### BACKGROUND

The principal components of biomass are cellulose and hemicellulose. Cellulose consists of polymers of β-1,4-linked glucose residues that are organized into higher order fibrillar structures. Hemicelluloses are heteropolysaccharides that include monosaccharides other than glucose, such as D-xylose, L-arabinose, D-mannose, D-glucose, D-galactose, and 4-O-methyl-D-glucuronic acid linked together not only by glycosidic linkages but also by ester linkages. The composition and structure of hemicellulose are more complicated than that of cellulose and can vary quantitatively and qualitatively in various woody plant species, grasses, and cereals.

Cellulose can be converted into sugars, such as glucose, and used as an energy source by numerous microorganisms including bacteria, yeast and fungi for industrial purposes. Cellulosic materials can also be converted into sugars by commercially available enzymes, and the resulting sugars can be used as a feedstock for industrial microorganisms to produce products such as plastics and ethanol. However, current cellulase products generally lack the ability to hydrolyze hemicellulosic materials, which remain unconsumed in the biomass compositions and may interfere with the handling and disposal of the biomass.

Accordingly, there remains a need to develop efficient enzyme systems for hydrolyzing both cellulose and hemicellulose, including the coproduction or blending of an optimized set of enzymes for converting hemicellulosic oligomers and polymers into free pentose for fermentation. Such optimized enzyme systems are desired to improve the efficiency and economics of biomass.

### SUMMARY

The present teachings provides optimized bioconverting enzyme blends, methods for producing the same, as well as methods of using the optimized bioconverting enzyme blend for converting biomass to sugar. The bioconverting enzyme blend comprises a mixture of a whole cellulase and one or more hemicellulases, the selection of which is dictated by the intended biomass substrate and processing conditions.

In one aspect, of the disclosure an enzyme blend composition for hydrolyzing a mixture of cellulosic and hemicellulosic materials is provided, comprising:
(a) a first enzyme composition comprising a cellulase,
(b) a second enzyme composition comprising at least one xylanase selected from a GH10 or GH11 xylanase, and
(c) a third enzyme composition comprising at least one additional hemicellulase that is not a GH10 or GH11 xylanase or not the same GH10 or GH11 xylanase as in (b),
wherein the enzyme blend composition provides at least one of (i) enhanced glucan conversion or (ii) enhanced xylan conversion compared to an equivalent enzyme blend composition lacking the at least one additional hemicellulase.

In some embodiments, the first enzyme composition is a whole cellulase blend from a filamentous fungus. In some embodiments, the first enzyme composition is a whole cellulase blend from a filamentous fungus supplemented with an addition amount of β-glucosidase.

In some aspects the second enzyme composition comprises xylanase XYN2 from *Trichoderma reesei.* In some aspects the second enzyme composition comprises xylanase XYN3 from *Trichoderma reesei.*

In some embodiments, the at least one xylanase has an amino acid sequence having at least 80% identity to an amino acid sequence selected from SEQ ID NO: 1 or SEQ ID NO:2. In some aspects the at least one xylanase has an amino acid sequence having at least 85%, at least 90%, at least 91%, at least 92%, at least 93%, at least 94%, at least 95%, at least 96%, at least 97%, at least 98%, or even at least 99% amino acid sequence identity to identity to an amino acid sequence selected from SEQ ID NO: 1 or SEQ ID NO: 2. In particular aspects the at least one xylanase has an amino acid sequence selected from SEQ ID NO: 1 or SEQ ID NO: 2.

In some aspects the at least one additional hemicellulase is selected from the group consisting of a GH54 hemicellulase, a GH62 hemicellulase, a GH27 hemicellulase, a GH36 hemicellulase, a GH5 hemicellulase, a GH74 hemicellulase, a GH67 hemicellulase, a GH28 hemicellulase, a GH11 hemicellulase, a GH10 hemicellulase, a GH3 hemicellulase, and a CE5 hemicellulase.

The at least one additional hemicellulase is a β-xylosidase and an arabinofuranosidase. The β-xylosidase is BXL1 from *Trichoderma reesei* and the arabinofuranosidase is ABF3 from *Trichoderma reesei.*

In some aspects the first enzyme composition is a whole cellulase blend from a filamentous fungus supplemented with an addition amount of β-glucosidase, the second enzyme composition comprises a xylanase, and the at least one additional hemicellulase is a combination of a β-xylosidase and arabinofuranosidase.

In some embodiments, the at least one additional hemicellulase is a *Trichoderma reesei* hemicellulase selected from the group consisting of α-arabinofuranosidase I (ABF1), α-arabinofuranosidase II (ABF2), α-arabinofuranosidase III (ABF3), α-galactosidase I (AGL1), α-galactosidase II (AGL2), α-galactosidase III (AGL3), acetyl xylan esterase I (AXE1), acetyl xylan esterase III (AXE3), endoglucanase VI (EG6), endoglucanase VIII (EG8), α-glucuronidase I (GLR1), β-mannanase (MAN1), polygalacturonase (PEC2), xylanase I (XYN1), xylanase II (XYN2), xylanase III (XYN3), and β-xylosidase (BXL1).

In some aspects the at least one additional hemicellulase has an amino acid sequence having at least 80% identity to an amino acid sequence selected from the group consisting of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17. In some aspects the at least one additional hemicellulase has an amino acid sequence having at least 85%, at least 90%, at least 91%, at least 92%, at least 93%, at least 94%, at least 95%, at least 96%, at least 97%, at least 98%, or even at least 99% amino acid sequence identity to one of the aforementioned amino acid sequences. In particular aspects the at least one additional hemicellulase has an amino acid sequence corresponding to one of aforementioned amino acid sequences.

In another aspect, a method for hydrolyzing a mixture of cellulosic and hemicellulosic materials is provided, comprising contacting the mixture of cellulosic and hemicellulosic materials with:
(a) a first enzyme composition comprising a cellulase,
(b) a second enzyme composition comprising at least one xylanase selected from a GH10 or GH11 xylanase, and
(c) a third enzyme composition comprising at least one additional hemicellulase that is not a GH10 or GH11 xylanase or not the same GH10 or GH11 xylanase as in (b),
thereby hydrolyzing the mixture of cellulosic and hemicellulosic materials,
wherein the contacting results in at least one of (i) enhanced glucan conversion or (ii) enhanced xylan conversion compared to equivalent contacting in the absence of the at least one additional hemicellulase.

In some aspects the first enzyme composition is a whole cellulase blend from a filamentous fungus. In some aspects the first enzyme composition is a whole cellulase blend from a filamentous fungus supplemented with an addition amount of β-glucosidase.

In some aspects the second enzyme composition comprises xylanase XYN2 from *Trichoderma reesei.* In some aspects the second enzyme composition comprises xylanase XYN3 from *Trichoderma reesei.*

In some aspects the at least one xylanase has an amino acid sequence having at least 80% identity to an amino acid sequence selected from SEQ ID NO: 1 or SEQ ID NO: 2. In some aspects the at least one xylanase has an amino acid sequence having at least 85%, at least 90%, at least 91%, at least 92%, at least 93%, at least 94%, at least 95%, at least 96%, at least 97%, at least 98%, or even at least 99% amino acid sequence identity to identity to an amino acid sequence selected from SEQ ID NO: 1 or SEQ ID NO: 2. In particular aspects the at least one xylanase has an amino acid sequence selected from SEQ ID NO: 1 or SEQ ID NO: 2.

In some aspects the at least one additional hemicellulase is selected from the group consisting of a GH54 hemicellulase, a GH62 hemicellulase, a GH27 hemicellulase, a GH36 hemicellulase, a GH5 hemicellulase, a GH74 hemicellulase, a GH67 hemicellulase, a GH28 hemicellulase, a GH11 hemicellulase, a GH10 hemicellulase, a GH3 hemicellulase, and a CE5 hemicellulase.

The at least one additional hemicellulase is a β-xyiosidaseand an arabinofuranosidase. The β-xylosidase is BXL1 from *Trichoderma reesei* and the arabinofuranosidase is ABF3 from *Trichoderma reesei.*

In some aspects the first enzyme composition is a whole cellulase blend from a filamentous fungus supplemented with an addition amount of β-glucosidase, the second enzyme composition comprises xylanase, and the at least one additional hemicellulase is a combination of a β-xylosidase and arabinofuranosidase.

In some aspects the at least one additional hemicellulase is a *Trichoderma reesei* hemicellulase selected from the group consisting of α-arabinofuranosidase I (ABF1), α-arabinofuranosidase II (ABF2), α-arabinofuranosidase III (ABF3), α-galactosidase I (AGL1), α-galactosidase II (AGL2), α-galactosidase III (AGL3), acetyl xylan esterase I (AXE1), acetyl xylan esterase III (AXE3), endoglucanase VI (EG6), endoglucanase VIII (EG8), α-glucuronidase I (GLR1), β-mannanase (MAN1), polygalacturonase (PEC2), xylanase I (XYN1), xylanase II (XYN2), xylanase III (XYN3), and β-xylosidase (BXL1).

In some aspects the at least one additional hemicellulase has an amino acid sequence having at least 80% identity to an amino acid sequence selected from the group consisting of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17. In some aspects the at least one additional hemicellulase has an amino acid sequence having at least 85%, at least 90%, at least 91%, at least 92%, at least 93%, at least 94%, at least 95%, at least 96%, at least 97%, at least 98%, or even at least 99% amino acid sequence identity to one of the aforementioned amino acid sequences. In particular aspects the at least one additional hemicellulase has an amino acid sequence corresponding to one of aforementioned amino acid sequences.

In some aspects contacting the mixture of cellulosic and hemicellulosic materials with the first enzyme composition, the second enzyme composition, and the third enzyme composition are performed simultaneously.

In some aspects the first enzyme composition, the second enzyme composition, and the third enzyme composition are provided in a single composition enzyme blend.

These and other aspects of the present compositions and methods will be apparent from the following description.

### DETAILED DESCRIPTION

### I. Definitions

Unless defined otherwise herein, all technical and scientific terms used herein have the same meaning as commonly understood by one of ordinary skill in the art. The headings provided herein are not limitations of the various aspects or embodiments of the invention described under one heading may apply to the compositions and methods as a whole. Both the foregoing general description and the following detailed description are exemplary and explanatory and are not restrictive of the compositions and methods described herein. The use of the singular includes the plural unless specifically stated otherwise, and the use of "or" means "and/or" unless state otherwise. The terms "comprise," "comprising," "comprises," "include," "including," and "includes" are not intended to be limiting. All patents and publications, including all amino acid and nucleotide sequences disclosed within such patents and publications, referred to herein are expressly incorporated by reference. The following terms are defined for clarity:

As used herein, the term "cellulose: refers a polysaccharide consisting of β(1→4) linked D-glucose units having the general formula (C₆H₁₀O₅)ₙ. Cellulose is the structural component of the primary cell wall of green plants, many forms of algae and the oomycetes.

As used herein, the term "cellulase" refers to an enzyme capable of hydrolyzing cellulose polymers to shorter oligomers and/or glucose.

As used herein, the term "whole cellulase composition/preparation/mixture" or the like refers to both naturally occurring and non-naturally occurring compositions that include a plurality of cellulases produced by an organism, for example a filamentous fungus. One example of a whole cellulase composition is medium in which filamentous fungi are cultured, which includes secreted cellulases, such as one or more cellobiohydrolases, one or more endoglucanases, and one or more β-glucosidases at a predetermined ratio.

As used herein, "hemicellulose" is a polymer component of plant materials that contains sugar monomers other than glucose, in contrast to cellulose, which contains only glucose. In addition to glucose, hemicellulose may include xylose, mannose, galactose, rhamnose, and arabinose, with xylose being the most common sugar monomer. Hemicelluloses contain most of the D-pentose sugars, and occasionally small amounts of L-sugars. The sugars in hemicellulose may be linked by ester linkages as well as glycosidic linkages. Exemplary forms of hemicellulose include but are not limited to are galactan, mannan, xylan, arabanan, arabinoxylan, glucomannan, galactomanan, and the like.

As used herein, the term "hemicellulase" refers to a class of enzymes capable of breaking hemicellulose into its component sugars or shorter polymers, and includes endo-acting hydrolases, exo-acting hydrolases, and various esterases.

As used herein, the term "xylanase" refers to a protein or polypeptide domain of a protein or polypeptide derived from a microorganism, *e.g*., a fungus, bacterium, or from a plant or animal, and that has the ability to catalyze cleavage of xylan at one or more of various positions of xylan's carbohydrate backbone, including branched xylans and xylooligosaccharides. Note that a xylanase is a type of hemicellulase.

As used herein, a "biomass substrate" is a material containing both cellulose and hemicellulose.

As used herein, a "naturally occurring" composition is one produced in nature or by an organism that occurs in nature.

As used herein, a "variant" protein differ from the "parent" protein from which it is derived by the substitution, deletion, or addition of a small number of amino acid residues, for example, 1, 2, 3, 4, 5, 6, 7, 8, 9, 10, 11, 12, 13, 14, 15, 16, 17, 18, 19, 20, or more amino acid residues. In some cases, the parent protein is a "wild-type," "native," or "naturally-occurring" polypeptides. Variant proteins may be described as having a certain percentage sequence identity with a parent protein, *e.g.*, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at even at least 99%, which can be determined using any suitable software program known in the art, for example those described in CURRENT PROTOCOLS IN MOLECULAR BIOLOGY (F. M. Ausubel et al. (eds) 1987, Supplement 30, section 7.7.18).

Preferred programs include the Vector NTI Advance^{™} 9.0 (Invitrogen Corp. Carlsbad, CA), GCG Pileup program, FASTA (Pearson et al. (1988) Proc. Natl, Acad. Sci USA 85:2444-2448), and BLAST (BLAST Manual, Altschul et al., Natl Cent. Biotechnol. Inf., Natl Lib. Med. (NCIB NLM NIH), Bethesda, Md., and Altschul et al. (1997) NAR 25:3389-3402). Another preferred alignment program is ALIGN Plus (Scientific and Educational Software, PA), preferably using default parameters. Another sequence software program that finds use is the TFASTA Data Searching Program available in the Sequence Software Package Version 6.0 (Genetics Computer Group, University of Wisconsin, Madison, WI).

### II. Bioconverting enzyme blend compositions and methods of use, thereof

Cellulose is a homopolymer of anhydrocellobiose and thus a linear β-(1-4)-D-glucan. In contrast, hemicelluloses include a variety of compounds, such as xylans, xyloglucans, arabinoxylans, and mannans, in complex branched structures, and with a spectrum of substituents. As a consequence of the complex branching and heterogenous composition of hemicelluloses, particularly arabinoxylans, the enzymatic degradation of plant material requires the action of a battery of both debranching and depolymerizing activities. Additionally, the degradation of plant materials requires enzymes that act on hemicelluloses containing both five-carbon sugars (pentoses), such as xylose and arabinose, and six-carbon sugars (hexoses), such as mannose and glucose.

Enzyme hydrolysis of hemicellulose to its monomers requires the participation of several hemicellulase enzymes with different functions. Hemicellulases can be placed into three general categories: endo-acting enzymes that attack internal bonds within the polysaccharide, exo-acting enzymes that act processively from either the reducing or nonreducing end of a polysaccharide chain, and the accessory enzymes, acetylesterases, and esterases that hydrolyze lignin glycoside bonds, such as coumaric acid esterase and ferulic acid esterase.

While certain fungi produce complete cellulase systems which include exo-cellobiohydrolases (or CBH-type cellulases), endoglucanases (or EG-type cellulases), and β-glucosidases (or BG-type cellulases), known cellulase enzymes and mixtures, thereof, typically have limited activity against hemicellulose, and limited value in hydrolyzing plant materials. The present bioconverting enzyme blend compositions and methods are based, in part, on the observation that certain combinations of cellulases and hemicellulases significantly increase the efficiency of plant material hydrolysis, primarily as determined by monitoring the conversion of glucan and xylan.

The exemplary cellulase composition used to identify cellulase/hemicellulase compositions that increase the hydrolysis of glucan and/or xylan is a whole cellulase compositions produced by a filamentous fungus (*i.e., Trichoderma reesei*). The composition includes several exo-cellobiohydrolases and endoglucanases, and is supplemented with additional β-glucosidase to increase the release of glucose. This composition is commercially available as ACCELLERASE 1000^{™} (Danisco A/S, Genencor Division, Palo Alto, CA). ACCELLERASE 1000^{™} includes exo-cellobiohydrolases (i.e., about 50% (wt/wt) CBHI (CEL7A) and about 14% CBHII (CEL6A), endoglucanases (i.e., about 12% EGI (CEL7B) and about 10% EGII (CEL5A)), and β-glucosidase (i.e., about 5% BGLI (CEL3A). A small amount of XYN2 (i.e., less than about 1%) may also be present. Other components that are not identified are also in amounts of less than about 1%.

Other cellulase compositions may be used, including other whole cellulase mixtures and cellulase mixtures assembled from multiple individually isolated cellulases. Preferred cellulase compositions include at least one each of an exo-cellobiohydrolase, an endoglucanase, and a β-glucosidase. In some embodiments, a whole broth that includes multiple cellulases is prepared from an organism such as an *Acremonium, Aspergillus, Emericella, Fusarium, Humicola, Mucor, Myceliophthora, Neurospora, Scytalidium, Thielavia, Tolypocladium, Penicillium,* or *Trichoderma* spp., or species derived therefrom.

The composition further includes, at least one, and in some cases two, three, or more hemicellulases. Examples of suitable additional hemicellulases include xylanases, arabinofuranosidases, acetyl xylan esterase, glucuronidases, endogalactanase, mannanases, endo or exo-arabinases, exo-galactanases, and mixtures thereof. Examples of suitable endo-acting hemicellulases include endo-arabinanase, endo-arabinogalactanase, endoglucanase, endo-mannanase, endo-xylanase, and feraxan endoxylanase. Examples of suitable exo-acting hemicellulases include α-L-arabinosidase, β-L-arabinosidase, α-1,2-L-fucosidase, α-D-galactosidase, β-D-galactosidase, β-D-glucosidase, β-D-glucuronidase, β-D-mannosidase, β-D-xylosidase, exo-glucosidase, exo-cellobiohydrolase, exo-mannobiohydrolase, exo-mannanase, exoxylanase, xylan α-glucuronidase, and coniferin β-glucosidase. Examples of suitable esterases include acetyl esterases (acetyl xylan esterase, acetylgalactan esterase, acetylmannan esterase, and acetylxylan esterase) and aryl esterases (coumaric acid esterase and ferulic acid esterase).

Preferably, the present compositions and methods include at least one xylanase, which is a particular type of hemicellulase that cleaves the xylan main chains of hemicellulose. Preferably, the xylanase is endo-1,4-β-xylanase (E.C. 3.2.1.8). Numerous xylanases from fungal and bacterial microorganisms have been identified and characterized (see, *e.g.,* U.S. Pat. No. 5,437,992; Coughlin, M. P. *supra;* Biely, P. et al. (1993) Proceedings of the second TRICEL symposium on Trichoderma reesei Cellulases and Other Hydrolases, Espoo 1993; Souminen, P. and Reinikainen, T. (eds)., in Foundation for Biotechnical and Industrial Fermentation Research 8:125-135). Three specific xylanases (XYN1, XYN2, and XYN3) have been identified in *T. reesei* (Tenkanen, et al. (1992) Enzyme Microb. Technol. 14:566; Torronen, et al. (1992) Bio/Technology 10:1461; and Xu, et al. (1998) Appl. Microbiol. Biotechnol. 49:718). A fourth xylanase (XYN4) isolated from *T. reesei* is described in U.S. Patent Nos. 6,555,335 and 6,768,001 to Saloheimo et al., entitled *Xylanase from Trichoderma reesei,* method for production thereof, and methods employing this enzyme, which is incorporated herein by reference in its entirety.

Exemplary xylanases for use in the present compositions and methods are XYN2 and XYN3. Suitable variants of XYN2 and XYN3, and suitable related enzymes from other organisms, have at least 80%, at least 85%, at least 90%, at least 91%, at least 92%, at least 93%, at least 94%, at least 95%, at least 96%, at least 97%, at least 98%, or even at least 99% amino acid sequence identity to of XYN2 or XYN3 (i.e., SEQ ID NOs: 1 and 2, respectively).

In addition to the cellulase composition and a xylanase, the compositions and methods may include one or more additional hemicellulases, such as an endo-acting hemicellulase, an exo-acting hemicellulase, and/or an esterases.

Suitable endo-acting hemicellulases include but are not limited to mannan endo-1,4-β-mannosidase (E.C. 3.2.1.78, also known as β-mannase and β-mannanase), which catalyzes the random endohydrolysis of 1,4,-β-D-mannosiic linkages in mannans, galactomannans, glucomannans; α-amylase (E.C. 3.2.1.1), which catalyzes the endohydrolysis of 1,4-α-D-glucosidic linkages in polysaccharides containing three or more 1,4-α-linked D-glucose units; xylan α-1,2-glucuronosidase (E.C. 3.2.1.131, also known as α-glucuronosidase), which catalyzes the hydrolysis of a-D-1,2-(4-O-methyl)glucuronosyl links in the main chain of hardwood xylans; and endoglucanase (E.C. 3.2.1.4), which catalyzes endohydrolysis of 1,4-β-D-glucosidc linkages in cellulase, lichenin, and cereal β-D-glucans. Multiple subtypes of endoglucanase have been identified and are suitable for use in the compositions and methods, for example, endoglucanase I, endoglucanase II, endoglucanase III, endoglucanase V, and endoglucanase VI.

Suitable exo-acting hemicellulases include but are not limited to α-arabinofuranosidase, α-galactosidase, and β-xylosidase. α-arabinofuranosidase, also known as a-*N*-arabinofuranosidase (E.C. 3.2.1.55), catalyzes the hydrolysis of terminal non-reducing α-L-arabinofuranoside residues in α-L-arabinosides. α -galactosidase (E.C. 3.2.1.22) catalyzes the hydrolysis of terminal, non-reducing α-D-galactose residues in α-D-galactosides including galactose oligosaccharides and galactomannans. Any of the three known subtypes, *i.e.*, α-galactosidase I (agI1), α-galactosidase II (agI2) and α-galactosidase III (agl3) can be used. Glucoamylase, also known as glucan 1,4-α-glucosidase (E.C. 3.2.1.3), catalyzes hydrolysis of terminal 1,4-linked α-D-glucose residues successively from non-reducing ends of the chains with release of β-D-glucose. β-glucosidase (E.C. 3.2.1.21) catalyzes the hydrolysis of terminal, non-reducing β-D-glucose residues with release of β-D-glucose. β-xylosidase, also known as xylan 1,4-β-xylosidase (E.C. 3.2.1.37), catalyzes hydrolysis of 1,4-β-D-xylans, to remove successive D-xylose residues from the non-reducing termini. Compositions that included a whole cellulase mixture, along with a xylanase and either an α-arabinofuranosidase or a β-xylosidase were particularly effective in glucan and/or xylan conversion.

Suitable esterases include but are not limited to ferulic acid esterase and acetyl xylan esterase. Ferulic acid esterase, also known as ferulate esterase (E.C. 3.1.1.73), catalyses the hydrolysis of the 4-hydroxy-3-methoxycinnamoyl (feruloyl) group from an esterified sugar, which is usually arabinose in "natural" substrates. Known microbial ferulic acid esterases are secreted into the culture medium. Any of the three known subtypes of ferulic acid esterase (fae1, fae2, and fae3) can be used in the present compositions and methods. Acetyl xylan esterase I (E.C. 3.1.1.72) catalyzes the deacetylation of xylans and xylo-oligosaccharides, and can also be used in the compositions and methods. U.S. Patent Nos. 5,426,043 and 5,681,732 to De Graaff et al. describe the cloning and expression of acetyl xylan esterases from fungal origin. EP 507 369 discloses a DNA sequence encoding an acetyl xylan esterase isolated from *Aspergillus niger.* U.S. Patent No. 5,830,734 to Christgau et al., entitled *Enzyme with acetyl esterase activity,* describes the isolation of a variety of esterases for use in the food industry.

In some embodiments, the at least one additional hemicellulase is selected from the group consisting of a GH54 hemicellulase, a GH62 hemicellulase, a GH27 hemicellulase, a GH36 hemicellulase, a GH5 hemicellulase, a GH74 hemicellulase, a GH67 hemicellulase, a GH28 hemicellulase, a GH11 hemicellulase, a GH10 hemicellulase, a GH3 hemicellulase, and a CE5 hemicellulase. In some embodiments, the at least one additional hemicellulase is selected from the group consisting of α-arabinofuranosidase I (ABF1), α-arabinofuranosidase II (ABF2), α-arabinofuranosidase III (ABF3), α-galactosidase I (AGL1), α-galactosidase II (AGL2), α-galactosidase III (AGL3), acetyl xylan esterase I (AXE1), acetyl xylan esterase III (AXE3), endoglucanase VI (EG6), endoglucanase VIII (EG8), α-glucuronidase I (GLR1), β-mannanase (MAN1), polygalacturonase (PEC2), xylanase I (XYN1), xylanase II (XYN2), xylanase III (XYN3), and β-xylosidase (BXL1), which may be from a filamentous fungus, such as *T. reesei.* In some embodiments, the at least one additional hemicellulase has an amino acid sequence having at least 80%, at least 85%, at least 90%, at least 91%, at least 92%, at least 93%, at least 94%, at least 95%, at least 96%, at least 97%, at least 98%, or even at least 99% amino acid sequence identity to an amino acid sequence selected from the group consisting of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17.

Variants of hemicellulases (including xylanases) may include substitutions, insertions, or deletions that do not substantially affect function, or add advantageous features to the enzymes. In some embodiments, the substitutions, insertions, or deletions are not in the conserved sequence motifs but are instead limited to amino acid sequences outside the conserved motifs. Exemplary substitutions are conservative substitutions, which preserve charge, hydrophobicity, or side group size relative to the parent amino acid sequence. Examples of conservative substitutions are provided in the following Table:

| Original Amino Acid Residue | Code | Acceptable Substitutions |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gin, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

It will be apparent that naturally occurring amino acids can be introduced into a polypeptide by changing the coding sequence of the nucleic acid encoding the polypeptide, while non-naturally-occurring amino acids are typically produced by chemically modifying an expressed polypeptide.

Further accessory enzymes, such as laccase (E.C. 1.10.3.2), which catalyzes oxidation of aromatic compounds, and consequent reduction of oxygen to water, can also be included in the bioconverting enzyme blends of the present compositions and methods.

In some embodiments, enzymes for use in the present bioconverting enzyme blends can be prepared from one or more strains of filamentous fungi. Suitable filamentous fungi include members of the subdivision Eumycota and Oomycota, including but are not limited to the following genera: *Aspergillus, Acremonium, Aureobasidium, Beauveria, Cephalosporium, Ceriporiopsis, Chaetomium, Chrysosporium, Claviceps, Cochiobolus, Cryptococcus, Cyathus, Endothia, Endothia mucor, Fusarium, Gilocladium, Humicola, Magnaporthe, Myceliophthora, Myrothecium, Mucor, Neurospora, Phanerochaete, Podospora, Paecilomyces, Pyricularia, Rhizomucor, Rhizopus, Schizophylum, Stagonospora, Talaromyces, Trichoderma, Thermomyces, Thermoascus, Thielavia, Tolypocladium, Trichophyton,* and *Trametes.* In some embodiments, the filamentous fungi include, but are not limited to the following: *A. nidulans, A. niger, A. awomari, A. aculeatus, A. kawachi e.g.,* NRRL 3112, ATCC 22342 (NRRL 3112), ATCC 44733, ATCC 14331 and strain UVK 143f, *A. oryzae, e.g.,* ATCC 11490, *N. crassa, Trichoderma reesei, e.g.,* NRRL 15709, ATCC 13631, 56764, 56765, 56466, 56767, and *Trichoderma viride, e.g.,* ATCC 32098 and 32086. In a preferred implementation, the filamentous fungi is a *Trichoderma* species. A particularly preferred species and strain for use in the present invention is *T. reesei* RL-P37.

In a particular embodiment, a single engineered strain overexpresses the component enzymes at the desired ratio so that no additional purification or supplementation is necessary. In an alternative embodiment, the bioconverting enzyme blend is obtained from two or more naturally occurring or engineered strains of filamentous fungi. The desired ratio of the component enzymes can be achieved by altering the relative amount of enzyme in the final blend. Even when two or more production strains are use, the desired ratio of component enzymes may be achieved by supplementation with purified or partially purified enzyme.

In particular embodiments, a hemicellulase is prepared from *Aspergillus aculeatus, Aspergillus awamori, Aspergillus foetidus, Aspergillus japonicus, Aspergillus nidulans, Aspergillus niger, or Aspergillus oryzae. In another aspect, whole broth is prepared from Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum* or *Fusarium verticilloides.* In another aspect, the hemicellulase complex is prepared from a *Humicola insolens, Humicola lanuginosa, Mucor miehei, Myceliophthora thermophila, Neurospora crassa, Scytalidium thermophilum,* or *Thielavia terrestris.* In other embodiments, a hemicellulase is prepared from a *Trichoderma harzianum, Trichoderma koningii, Trichoderma longibrachiatum, Trichoderma reesei, e.g.,* RL-P37 [Sheir-Neiss et al. (1984) Appl. Microbiol. Biotechnology 20:46-53; U.S. Pat. No. 4,797,361; available as a biologically pure culture from the permanent collection of the Northern Regional Research Laboratory, U.S. Department of Agriculture, Peoria, III., U.S.A. (NRRL 15709); ATCC 13631, 56764, 56466, 56767], or *Trichoderma viride e.g.,* ATCC 32098 and 32086.

In some embodiments, a component hemicellulase enzyme is produced by expressing a gene encoding the hemicellulase enzyme. For example, xylanase can be secreted into the extracellular space of, *e.g.,* a Gram-positive organism, such as *Bacillus* or *Actinomycetes,* or a eukaryotic organism, such as *Trichoderma, Aspergillus, Saccharomyces,* or *Pichia.* It is to be understood, that in some embodiments, one or more hemicellulase enzymes can be over-expressed in a recombinant microorganism relative to the native levels. The host cell may be genetically modified to reduce expression of one or more proteins that are endogenous to the cell. In one embodiment, the cell may contain one or more native genes, particularly genes that encode secreted proteins that have been deleted or inactivated. For example, one or more protease-encoding genes (*e.g.,* an aspartyl protease-encoding gene; see Berka et al. (1990) Gene 86:153-162 and U.S. Pat. No. 6,509,171), or cellulase-encoding genes, may be deleted or inactivated. The nucleic acids encoding the hemicellulase may be present in the genome of an organism or carried in a plasmid that replicates in the organism. Where the hemicellulase is expressed from the genome, the gene and regulator sequences associate therewith, can be introduced into the genome by random or homologous integration. In certain cases, *e.g.*, when a particularly high level of expression is desired, both random and homologous integration can be used.

The biomass substrate for use as a source of cellulose and hemicellulose for hydrolysis using the present enzyme compositions and methods can be, *e.g.,* herbaceous material, agricultural residues, forestry residues, municipal solid waste, waste paper, and pulp and paper residues, and the like. Common forms of biomass substrate include, but are not limited to trees, shrubs and grasses, wheat, wheat straw, sugar cane bagasse, corn, corn husks, corn kernel including fiber from kernels, products and by-products from milling of grains such as corn (including wet milling and dry milling) as well as municipal solid waste, waste paper and yard waste. The biomass substrate may be obtained from "virgin biomass" (such as trees, bushes, grasses, fruits, flowers, herbaceous crops, hard and soft woods.), "non-virgin biomass" (such as agricultural byproducts, commercial organic waste, construction and demolition debris, municipal solid waste and yard waste), or "blended biomass," which is a mixture of virgin and non-virgin biomass. The biomass substrate may include, *e.g.*, wood, wood pulp, papermaking sludge, paper pulp waste streams, particle board, corn stover, corn fiber, rice, paper and pulp processing waste, woody or herbaceous plants, fruit pulp, vegetable pulp, pumice, distillers grain, grasses, rice hulls, sugar cane bagasse, cotton, jute, hemp, flax, bamboo, sisal, abaca, straw, corn cobs, distillers grains, leaves, wheat straw, coconut hair, algae, switchgrass, and mixtures thereof.

The biomass substrate can be used directly or may be subjected to pretreatment using conventional methods known in the art. Such pretreatments include chemical, physical, and biological pretreatments. For example, physical pretreatment techniques include, without limitation, various types of milling, crushing, steaming/steam explosion, irradiation and hydrothermolysis. Chemical pretreatment techniques include, without limitation, dilute acid, alkaline agents, organic solvents, ammonia, sulfur dioxide, carbon dioxide, and pH-controlled hydrothermolysis. Biological pretreatment techniques include, without limitation, applying lignin-solubilizing microorganisms.

Optimum dosage levels of bioconverting enzyme blend, and cellulases and hemicellulases, therein, vary depending on the biomass substrate and the pretreatment technologies used. Operating conditions such as pH, temperature and reaction time may also affect rates of ethanol production. Preferably, the reactive composition contains 0.1 to 200 mg bioconverting enzyme blend per gram of biomass, more preferably 1 to 100 mg bioconverting enzyme blend per gram of biomass and most preferably 10-50 mg bioconverting enzyme blend per gram of biomass. Exemplary amounts are 0.1-50, 1-40, 20-40, 1-30, 2-40, and 10-20 mg bioconverting enzyme blend per gram of biomass. Alternatively, the amount of enzyme can be determined based on the amount of substrate in the system. In such a case, the reactive composition preferably contains 0.1 to 50 mg bioconverting enzyme blend per gram of total saccharides, more preferably, 1 to 30 mg bioconverting enzyme blend per gram of total saccharides, and more preferably 10 to 20 mg bioconverting enzyme blend per gram of total saccharides. Alternatively, the amount of enzyme can be determined based on the amount of cellulose substrate in the system. In such a case, the reactive composition preferably contains 0.2 to 100 mg bioconverting enzyme blend per gram of total glucan, more preferably, 2 to 60 mg bioconverting enzyme blend per gram of total glucan, and more preferably 20 to 40 mg bioconverting enzyme blend per gram of total glucan. Similarly, the amount of bioconverting enzyme blend utilized can be determined by the amount of hemicellulose in the substrate biomass. Accordingly, the reactive composition preferably contains 0.2 to 100 mg bioconverting enzyme blend per gram of hemicellulose, more preferably, 2 to 60 mg bioconverting enzyme blend per gram of hemicellulose, and more preferably 20 to 40 mg bioconverting enzyme blend per gram of hemicellulose.

In some embodiments, the present composition is in the form of a hemicellulose-enhanced whole cellulase composition, comprising a whole cellulase preparation and at least one hemicellulase, wherein the amount of hemicellulase is in the range of 1% to 50% of the total protein and the whole cellulase is in the range of less than 99% to 50% of total protein. For example, the hemicellulase may represent 1% of the total protein and the whole cellulase composition may represent 99% of the total protein, the hemicellulase may represent 2% of the total protein and the whole cellulase composition may represent 98% of the total protein, the hemicellulase may represent 3% of the total protein and the whole cellulase composition may represent 97% of the total protein, the hemicellulase may represent 4% of the total protein and the whole cellulase composition may represent 96% of the total protein, the hemicellulase may represent 5% of the total protein and the whole cellulase composition may represent 95% of the total protein, the hemicellulase may represent 6% of the total protein and the whole cellulase composition may represent 94% of the total protein, the hemicellulase may represent 7% of the total protein and the whole cellulase composition may represent 93% of the total protein, the hemicellulase may represent 8% of the total protein and the whole cellulase composition may represent 92% of the total protein, the hemicellulase may represent 9% of the total protein and the whole cellulase composition may represent 91% of the total protein, the hemicellulase may represent 10% of the total protein and the whole cellulase composition may represent 90% of the total protein, the hemicellulase may represent 11% of the total protein and the whole cellulase composition may represent 89% of the total protein, the hemicellulase may represent 12% of the total protein and the whole cellulase composition may represent 88% of the total protein, the hemicellulase may represent 13% of the total protein and the whole cellulase composition may represent 87% of the total protein, the hemicellulase may represent 14% of the total protein and the whole cellulase composition may represent 86% of the total protein, the hemicellulase may represent 15% of the total protein and the whole cellulase composition may represent 85% of the total protein, the hemicellulase may represent 16% of the total protein and the whole cellulase composition may represent 84% of the total protein, the hemicellulase may represent 17% of the total protein and the whole cellulase composition may represent 83% of the total protein, the hemicellulase may represent 18% of the total protein and the whole cellulase composition may represent 82% of the total protein, the hemicellulase may represent 19% of the total protein and the whole cellulase composition may represent 81% of the total protein, the hemicellulase may represent 20% of the total protein and the whole cellulase composition may represent 80% of the total protein, the hemicellulase may represent 21% of the total protein and the whole cellulase composition may represent 79% of the total protein, the hemicellulase may represent 22% of the total protein and the whole cellulase composition may represent 78% of the total protein, the hemicellulase may represent 23% of the total protein and the whole cellulase composition may represent 77% of the total protein, the hemicellulase may represent 24% of the total protein and the whole cellulase composition may represent 76% of the total protein, the hemicellulase may represent 25% of the total protein and the whole cellulase composition may represent 75% of the total protein, the hemicellulase may represent 26% of the total protein and the whole cellulase composition may represent 74% of the total protein, the hemicellulase may represent 27% of the total protein and the whole cellulase composition may represent 73% of the total protein, the hemicellulase may represent 28% of the total protein and the whole cellulase composition may represent 72% of the total protein, the hemicellulase may represent 29% of the total protein and the whole cellulase composition may represent 71% of the total protein, the hemicellulase may represent 30% of the total protein and the whole cellulase composition may represent 70% of the total protein, the hemicellulase may represent 31% of the total protein and the whole cellulase composition may represent 69% of the total protein, the hemicellulase may represent 32% of the total protein and the whole cellulase composition may represent 68% of the total protein, the hemicellulase may represent 33% of the total protein and the whole cellulase composition may represent 67% of the total protein, the hemicellulase may represent 34% of the total protein and the whole cellulase composition may represent 66% of the total protein, the hemicellulase may represent 35% of the total protein and the whole cellulase composition may represent 65% of the total protein, the hemicellulase may represent 36% of the total protein and the whole cellulase composition may represent 64% of the total protein, the hemicellulase may represent 37% of the total protein and the whole cellulase composition may represent 63% of the total protein, the hemicellulase may represent 38% of the total protein and the whole cellulase composition may represent 62% of the total protein, the hemicellulase may represent 39% of the total protein and the whole cellulase composition may represent 61% of the total protein, the hemicellulase may represent 40% of the total protein and the whole cellulase composition may represent 60% of the total protein, the hemicellulase may represent 41% of the total protein and the whole cellulase composition may represent 59% of the total protein, the hemicellulase may represent 42% of the total protein and the whole cellulase composition may represent 58% of the total protein, the hemicellulase may represent 43% of the total protein and the whole cellulase composition may represent 57% of the total protein, the hemicellulase may represent 44% of the total protein and the whole cellulase composition may represent 56% of the total protein, the hemicellulase may represent 45% of the total protein and the whole cellulase composition may represent 55% of the total protein, the hemicellulase may represent 46% of the total protein and the whole cellulase composition may represent 54% of the total protein, the hemicellulase may represent 47% of the total protein and the whole cellulase composition may represent 53% of the total protein, the hemicellulase may represent 48% of the total protein and the whole cellulase composition may represent 52% of the total protein, the hemicellulase may represent 49% of the total protein and the whole cellulase composition may represent 51% of the total protein, or the hemicellulase may represent 50% of the total protein and the whole cellulase composition may represent 50% of the total protein.

In use, the bioconverting enzyme blend compositions may be added to a suitable substrate material individually, *i.e*., as separate enzyme compositions, or as a single enzyme mixtures in which all cellulases and hemicellulases are present prior to addition to the substrate. Where the cellulases and hemicellulases are separate enzyme compositions, they may be added sequentially or simultaneously to the substrate. Where the cellulases and hemicellulases are present in a single mixture, they are added simultaneously.

Other aspects and embodiments of the compositions and method may be further understood in view of the following examples, which should not be construed as limiting. It will be apparent to those skilled in the art that many modifications, both to materials and methods, may be made without departing from the present teachings.

### EXAMPLES

ACCELLERASE 1000^{™} (Danisco A/S, Genencor Division, Palo Alto, CA), a whole broth of killed cellular material that includes a *T. reesei* whole cellulase mixture supplemented with *T. reesei* BGLU1 β-glucosidase, was used as source of cellulases. MULTIFECT^{®} Xylanase (Danisco A/S, Genencor Division, Palo Alto, CA), a xylanase II, high pl, formulated product, was used as a source of XYN2.

*T. reesei* hemicellulases were individually over-expressed in a strain of *T. reesei* in which the genes encoding CBHI, CBHII, EG1, and EG2 were deleted, to avoid the presence of these cellulases in the resulting cellular material (*e.g*., conditioned media or "broths"). Hemicellulases of interest ranged from <10% to 85% of total protein in these broths. In many cases, the broths were used directly; however, several hemicellulases were further purified to demonstrate that the observed activities were not the result of other protein present in the broth.

The acronyms, polypeptide SEQ ID NOs, and Carbohydrate-Active enZymes (CAZY) family and clan designations (where known) of the particular enzymes are provided in Table 1. The aforementioned XYN2 polypeptide has the amino acid sequence of SEQ ID NO: 1 and is a family GH11 Clan C enzyme. The amino acid sequences of the immature polypeptides are also shown, below.

**TABLE 1**

| Acronym | Enzyme | SEQ ID | Family | Clan |
|---|---|---|---|---|
| ABF1 | α-arabinofuranosidase I | 3 | GH54 | |
| ABF2 | α-arabinofuranosidase II | 4 | GH62 | F |
| ABF3 | α-arabinofuranosidase III | 5 | GH54 | |
| AGL1 | α-galactosidase I | 6 | GH27 | D |
| AGL2 | α-galactosidase II | 7 | GH36 | D |
| AGL3 | α-galactosidase III | 8 | GH27 | D |
| AXE1 | acetyl xylan esterase I | 9 | CE5 | |
| AXE3 | acetyl xylan esterase III | 10 | CE5 | |
| EG6 | endoglucanase VI | 11 | GH74 | |
| EG8 | endoglucanase VIII | 12 | GH5 | A |
| GLR1 | α-glucuronidase I | 13 | GH67 | |
| MAN1 | β-mannanase | 14 | GH5 | A |
| PEC2 | polyqalacturonase | 15 | GH28 | N |
| XYN1 | xylanase I | 16 | GH11 | C |
| XYN3 | xylanase III | 2 | GH10 | A |
| BXL1 | β-xylosidase | 17 | GH3 | |

XYN2 (SEQ ID NO: 1) 
MVSFTSLLAASPPSRASCRPAAEVESVAVEKRQTIQPGTGYNNGYFYSYWNDGHGGVTYTNG
PGGQFSVNWSNSGNFVGGKGWQPGTKNKVINFSGSYNPNGNSYLSVYGWSRNPLIEYYIVEN
FGTYNPSTGATKLGEVTSDGSVYDIYRTQRVNQPSIIGTATFYQYWSVRRNHRSSGSVNTANH
FNAWAQQGLTLGTMDYQIVAVEGYFSSGSASITVS
XYN3 (SEQ ID NO: 2) 
MKANVILCLLAPLVAALPTETIHLDPELAALRANLTERTADLWDRQASQSIDQLIKRKGKLYFGTA
TDRGLLQREKNAAIIQADLGQVTPENSMKWQSLENNQGQLNWGDADYLVNFAQQNGKSIRGH
TLIWHSQLPAWVNNINNADTLRQVIRTHVSTVVGRYKGKIRAWDVVNEIFNEDGTLRSSVFSRL
LGEEFVSIAFRAARDADPSARLYINDYNLDRANYGKVNGLKTYVSKWISQGVPIDGIGSQSHLS
GGGGSGTLGALQQLATVPVTELAITELDIQGAPTTDYTQVVQACLSVSKCVGITVWGISDKDSW
RASTNPLLFDANFNPKPAYNSIVGILQ
ABF1 (SEQ ID NO: 3) 
MLSNARIIAAGCIAAGSLVAAGPCDIYSSGGTPCVAAHSTTRALFSAYTGPLYQVKRGSDGATT
AISPLSSGVANAAAQDAFCAGTTCLITIIYDQSGRGNHLTQAPPGGFSGPESNGYDNLASAIGA
PVTLNGQKAYGVFVSPGTGYRNNAASGTAKGDAAEGMYAVLDGTHYNGACCFDYGNAETNS
RDTGNGHMEAIYFGDSTVWGTGSGKGPWIMADLENGLFSGSSPGNNAGDPSISYRFVTAAIK
GQPNQWAIRGGNAASGSLSTFYSGARPQVSGYNPMSKEGAIILGIGGDNSNGAQGTFYEGVM
TSGYPSDATENSVQANIVAARYAVAPLTSGPALTVGSSISLRATTACCTTRYIAHSGSTVNTQVV
SSSSATALKQQASWTVRAGLANNACFSFESRDTSGSYIRHSNFGLVLNANDGSKLFAEDATFC
TQAGINGQGSSIRSWSYPTRYFRHYNNTLYIASNGGVHVFDATAAFNDDVSFVVSGGFA
ABF2 (SEQ ID NO: 4) 
MELKALSAVVLSFVTLVAAAPATCTLPSTYRWNSTGALASPKSGWVSLKDFSHVIYNGQHLVW
GSTHDTGTIWGSMNFGLFSDWSNMATASQNKMTPGTVAPTVFYFAPKNIWVLAYQWGPTTFS
YLTSSNPSSVNGWSSPQPLFSGSISGSSPLDQTVIGDSTNMYLFFAGDDGKIYRASMPIGNFPG
SFGSTSTVVLSDERNNLFEAVQVYTVSGQKQYLMIVEAIGANGRYFRSFTATNLGGTWTPQAT
SESQPFAGKANSGATWTNDISHGDLIRSNPDQTMTIDPCNLQFLYQGRATNSGGDYGLLPYRP
GLLTLQR
ABF3 (SEQ ID NO: 5) 
MSPRTDRRRSGLLALGLVAASPLATAGPCDIYASGGTPCVAAHSTTRALYGAYSGPLYQVSRG
SDGATTNIATLSAGGVANAAAQDSFCAGTTCLITVIYDQSGRGNHLTQAPPGGAASGPQPNGY
DNLASAIGAPVRLNGQKAYGVFIAPFTGYRNNQPNGTATGDQPQGMYAIFDGTHYNTGCCFD
YGNAETNSLDTGNGHMEAIYFGTGDGSGRGTGSGSGPWIMADLENGLFSGYDPINNPADPTI
NFRFVTAVVKGEPGQWAIRGGDATSGTLSTFYSGQRPANGYNPMSKEGAIILGIGGDNSNRAQ
GTFYEGVMTSGYPSDSTENAVQANLVAAKYVYDTSLMTSGPALSVGSSISLRATTSCCTNRYIA
HTGATVNTQVVSSSSSTALKQQASWTVRTGLGNSACFSFESRDSPGSFIRHSNYQLMVNAND
NSKLFHEDATFCPQAGLNGQGNSFRSWSYPTRYWRHFNSLGYIAANGGEHDFDTTTLFNDDV
SFVVSAGFA
AGL1 (SEQ ID NO: 6) 
MTPHSIDRAARPSVWSGLALLLSTAHAIVMPDGVTGKVPSLGWNSWNAYHCDIDESKFLSAAE
VIVSSGLLDAGYNYVNIDDCWSMKDGRVDGHIAVNTTRFPDGIDGLAKKVHDLGLKLGIYSTAG
TATCAGYPASLGYEDVDAADFADWGVDYLKYDNCNVPSDWQDEYVACAPDAVQTGPNGTCS
TALEPNLAPPGYDWSTSKSAERFNAMRNALAKQSREIVLSLCIWGVADVFSWGNETGISWRM
SGDISPEWGSVTHIINMNSFKMNSVGFWGHNDADILEVGNGNLTAAETRTHFALWAAMKSPLLI
GTDLAQLSQENIELLKNKHLLAFNQDSVYGQPATPYKWGVNPDWTFNYTNPAEYWAGPSSKG
HLVLMMNTLDHTVRKEAKWSEIPGLSAGRYEVRDVWTDKSLGCLSSYKTAVAAHDTAVILVGK
KCRNW
AGL2 (SEQ ID NO: 7) 
MLGAPSPRRLADVLAVTAGLVASVRAASPISVSGKSFALNGDNVSYRFHVDDDSKDLIGDHFG
GPATEDGVFPPIIGPIQGWVDLIGRQRREFPDLGRGDFRTPAVHIRQAAGYTVSDFQYKSHRVV
EGKPALRGLPSTFGDAGDVSTLVVHMYDNYSSVAADLTYSIFPKYDAIVRSVNITNMGKGNITIE
KLASLSVDLPYEDFDMLELKGDWAREGKRLRRKVDYGSQGFGSTTGYSSHLHNPFFSLITPTT
TESQGEAWGFSLVYTGSFSVEVEKGSQGLTRAAIGVNPYQLSWPLGPGETFSSPEAVAVFSTT
GVGGMSRKFHNLYRKHLIKSKFATQMHPVLLNSWEGLGFDYNDTTILHLAQESADLGIKLFVLD
DGWFGVKHPRVSDNAGLGDWEANPKRFPQGLPDFISDVTKLKVANSSDHLQFGLWFEPEMV
NPNSTLYMEHPDWAIHAGSYPRTLTRNQLVLNVALPEVQDFIIESLSNILSNASISYVKWDNNRG
IHEAPYPGLDYAYMLGLYRVFDTLSSKFPNVRWEGCASGGGRFDPGVLQYFPHIWTSDDTDA
VERIAIQFGTSLVYPPSAMGAHVSAVPNGQTQRTTSIAFRAHVAMMGGSFGFELTPAEMPEDD
KAQIPGIIALAEKVNPIVVKGDMWRLSLPEESNWPAALFISQDGSQAVLFYFQIRANINNAWPVL
RLQGLDASAKYKIDGNQTFSGATLMNIGLQYQFNGDYDSKVVFLEKQT
AGL3 (SEQ ID NO: 8) 
MSPSAAVLIPLAAAVLLRPVVGQTQCGGNLYTPGTLNFTLECYNAFQDCVAQFEANASQVDCN
DGKGNLFMQQQANLGASPGSQNNDAIIAFQDIRDLCLLSGSTTATWGYSDNQWYWAAAEDAC
YTNDPTRTDVVKTHPAPFCIQNRDSSLPECYPQPDATPPGGPLKVIKTAKTRNGFKSSARGWN
TYGVQALVNGSQVVPSFAGQSGLFYTQKFVETQCGVLARPEFKKAGYDLCSLDSGWQATTAV
DQHGRIIYNTTRFNLPELASWLHKRDLKLGVYITPGVPCLAHNQTILGTNIKIKDVLNGNNDQINC
DFDFRKDGVQQWHDSVVAQWASWGVDMLKLDFLTPGSPSNGANLACDSSDAVRAYQKAIKK
SGRKIRLDISWKLCRNETWLPIWSDLAESMRTDQDLDNYGTNTLMAWQVGQRAIENYRQYIGL
QAQRNVPLTIYPDMDALFTVNPEHLAGVNDTIRYTVQNHWLGAGANLIIGGDMEQVDALGLKLT
TSKQSIDAADFFAKYPMQPRNPGTGSNAAKQLQAWIGGPSDDHEAYVLIVNYGPDLGNGGFS
TKLYGKQKVTVSLKDLGISGSAWTFTDIWSGKSSRVTGSYSAWLTEGESQLLRLKRTH
AXE1 (SEQ ID NO: 9) 
MPSVKETLTLLLSQAFLATGSPVDGETVVKRQCPAIHVFGARETTVSQGYGSSATVVNLVIQAH
PGTTSEAIVYPACGGQASCGGISYANSVVNGTNAAAAAINNFHNSCPDTQLVLVGYSQGAQIF
DNALCGGGDPGEGITNTAVPLTAGAVSAVKAAIFMGDPRNIHGLPYNVGTCTTQGFDARPAGF
VCPSASKIKSYCDAADPYCCTGNDPNVHQGYGQEYGQQALAFINSQLSSGGSQPPGGGPTST
SRPTSTRTGSSPGPTQTHWGQCGGQGWTGPTQCESGTTCQVISQWYSQCL
AXE3 (SEQ ID NO: 10) 
MPSIKSTVTFLLSQALLATATPMDLEKRQCPGIHVFGARETTAPPGYGSSATVVNLIINAHPGTT
AEAINYPACGGQAQCGGISYANSVVAGINAVVQAVTNFHNRCPSTKLVLVGYSQGGQIMDDAL
CGGGDPAEGYPNTAVPLPAAAVSAIRAAIFMGDPRYVHGLAYNVGSCQAQGFAPRNVGFVCP
SGNKIKSYCDASDPYCCNGNNANTHQGYGQEYGQQALAFVNSLLG
EG6 (SEQ ID NO: 11) 
MKVSRVLALVLGAVIPAHAAFSWKNVKLGGGGGFVPGIIFHPKTKGVAYARTDIGGLYRLNADD
SWTAVTDGIADNAGWHNWGIDAVALDPQDDQKVYAAVGMYTNSWDPSNGAIIRSSDRGATW
SFTNLPFKVGGNMPGRGAGERLAVDPANSNIIYFGARSGNGLWKSTDGGVTFSKVSSFTATGT
YIPDPSDSNGYNSDKQGLMWVTFDSTSSTTGGATSRIFVGTADNITASVYVSTNAGSTWSAVP
GQPGKYFPHKAKLQPAEKALYLTYSDGTGPYDGTLGSVWRYDIAGGTWKDITPVSGSDLYFGF
GGLGLDLQKPGTLVVASLNSWWPDAQLFRSTDSGTTWSPIWAWASYPTETYYYSISTPKAPWI
KNNFIDVTSESPSDGLIKRLGWMIESLEIDPTDSNHWLYGTGMTIFGGHDLTNWDTRHNVSIQS
LADGIEEFSVQDLASAPGGSELLAAVGDDNGFTFASRNDLGTSPQTVWATPTWATSTSVDYA
GNSVKSVVRVGNTAGTQQVAISSDGGATWSIDYAADTSMNGGTVAYSADGDTILWSTASSGV
QRSQFQGSFASVSSLPAGAVIASDKKTNSVFYAGSGSTFYVSKDTGSSFTRGPKLGSAGTIRDI
AAHPTTAGTLYVSTDVGIFRSTDSGTTFGQVSTALTNTYQIALGVGSGSNWNLYAFGTGPSGA
RLYASGDSGASWTDIQGSQGFGSIDSTKVAGSGSTAGQVYVGTNGRGVFYAQGTVGGGTGG
TSSSTKQSSSSTSSASSSTTLRSSVVSTTRASTVTSSRTSSAAGPTGSGVAGHYAQCGGIGWT
GPTQCVAPYVCQKQNDYYYQCV
EG8 (SEQ ID NO: 12) 
MRATSLLAAALAVAGDALAGKIKYLGVAIPGIDFGCDIDGSCPTDTSSVPLLSYKGGDGAGQMK
HFAEDDGLNVFRISATWQFVLNNTVDGKLDELNWGSYNKVVNACLETGAYCMIDMHNFARYN
GGIIGQGGVSDDIFVDLWVQIAKYYEDNDKIIFGLMNEPHDLDIEIWAQTCQKVVTAIRKAGATS
QMILLPGTNFASVETYVSTGSAEALGKITNPDGSTDLLYFDVHKYLDINNSGSHAECTTDNVDA
FNDFADWLRQNKRQAIISETGASMEPSCMTAFCAQNKAISENSDVYIGFVGWGAGSFDTSYILT
LTPLGKPGNYTDNKLMNECILDQFTLDEKYRPTPTSISTAAEETATATATSDGDAPSTTKPIFRE
ETASPTPNAVTKPSPDTSDSSDDDKDSAASMSAQGLTGTVLFTVAALGYMLVAF
GLR1 (SEQ ID NO: 13) 
MVIRSLLLLLLAAIVPVFAESGIDAWLRYARLPSSATRGHLTSFPDRIVVLNASKNGPLASASSEL
HKGIKGILGLDLDVSSRGGKHCSTQKSIVISTLDTYQSACGKLSPKLNLKEDGYWLSTKGGSVQI
IGQNERGALYGAFQYLSYLGQGDFSGKAFASNPSAPVRWSNQWDNLNAATAAHGSIERGYG
GPSIFFENGLIKEDLSRVPLYGRLLASVGLNGIVINNVNADANLLNETNLQGLKRIADLFRPWGV
NVGISLNFASPQVLGDLSTFDPLDDSVIKWWTDKTDRIYQLVPDLAGYLVKANSEGQPGPLTYN
RTLAEGANLFAKAVQPHGGIVVFRAFVYDQLNETDWKADRANAAVDFFKSLDGQFDDNVLVQI
KYGPIDFQVREPASPLFANLPKTAVSIELEVTQEYLGQQSHLVYLPPLWQTVLGFDMRYNNRQ
SYVRDIISGEVFGHKLGGYAGVINVGMDDTWLGSHLAMSNMFAYGRLAWNPRADSRDIVEEW
TRLTFGLDRDVVSTIADMSLKSWPAYEGYSGNLGIQTLTDILYTHYGANPASQDNNGWGQWT
RADSKTIGMDRTVSNGTGNAGQYPKEVAARFEHTQTTPDDLMLWFHHVPYTFRLHSGKSVIQ
HFYDAHYTGAATVQRFPAAWKSLKSKIDTERYNAVLYKLQYQTGHSLVWRDAITEFYRNLSSIP
DQLNRVRNHPHRIEAEDMDLSGFTVVNVSPTECASKYKAIATNGTGTATTRLNVPSGKYTVAV
NYYDVINGTASYDVLLNGKSLGKWKGDSETHLGHDFSTFLDCHSAIRITFEGVRISRGDKLTIRG
TGNAQEQAAIDYVSILPQGVVD
MAN1 (SEQ ID NO: 14) 
MMMLSKSLLSAATAASALAAVLQPVPRASSFVTISGTQFNIDGKVGYFAGTNCYWCSFLTNHA
DVDSTFSHISSSGLKVVRVWGFNDVNTQPSPGQIWFQKLSATGSTINTGADGLQTLDYVVQSA
EQHNLKLIIPFVNNWSDYGGINAYVNAFGGNATTWYTNTAAQTQYRKYVQAVVSRYANSTAIFA
WELGNEPRCNGCSTDVIVQWATSVSQYVKSLDSNHLVTLGDEGLGLSTGDGAYPYTYGEGTD
FAKNVQIKSLDFGTFHLYPDSWGTNYTWGNGWIQTHAAACLAAGKPCVFEEYGAQQNPCTNE
APWQTTSLTTRGMGGDMFWQWGDTFANGAQSNSDPYTVWYNSSNWQCLVKNHVDAINGG
TTTPPPVSSTTTTSSRTSSTPPPPGGSCSPLYGQCGGSGYTGPTCCAQGTCIYSNYWYSQCL
NT
PEC2 (SEQ ID NO: 15) 
MLKLSLFLGAVTASLCVQAHAVPPPTVTQAPKLEDRATTCTFSGSNGASSASKSQKSCATIVLS
NVAVPSGVTLDLSDLNDGTTVIFEGTTTWGYKEWSGPLLQIEGNDITIQGASGAVLNPDGARW
WDGQGGNGGKTKPKFFAAHDLTSSSITNLYIKNTPVQAVSVNGVNGLTITGMTIDNSAGDSGG
GHNTDGFDIGSSSNVVISGAKVYNQDDCVAVNSGTNITFTGGLCSGGHGLSIGSVGGRDDNTV
QTVTFSNSQVTKSANGIRIKATAGKTGTIKGVTYTGITLSSITGYGILIEQNYDGGDLHGSPTSGIP
ITNLVLQNISGSNGVVSSGNNIAIVCGSGACSNWTWSNVVVTGGKKYGSCQNVPSPATC
XYN1 (SEQ ID NO: 16) 
MVAFSSLICALTSIASTLAMPTGLEPESSVNVTERGMYDFVLGAHNDHRRRASINYDQNYQTG
GQVSYSPSNTGFSVNWNTQDDFVVGVGWTTGSSAPINFGGSFSVNSGTGLLSVYGWSTNPL
VEYYIMEDNHNYPAQGTVKGTVTSDGATYTIWENTRVNEPSIQGTATFNQYISVRNSPRTSGTV
TVQNHFNAWASLGLHLGQMNYQVVAVEGWGGSGSASQSVSN
BXL1 (SEQ ID NO: 17)

MVNNAALLAALSALLPTALAQNNQTYANYSAQGQPDLYPETLATLTLSFPDCEHGPLKNNLVC
DSSAGYVERAQALISLFTLEELILNTQNSGPGVPRLGLPNYQVWNEALHGLDRANFATKGGQF
EWATSFPMPILTTAALNRTLIHQIADIISTQARAFSNSGRYGLDVYAPNVNGFRSPLWGRGQET
PGEDAFFLSSAYTYEYITGIQGGVDPEHLKVAATVKHFAGYDLENWNNQSRLGFDAIITQQDLS
EYYTPQFLAAARYAKSRSLMCAYNSVNGVPSCANSFFLQTLLRESWGFPEWGYVSSDCDAVY
NVFNPHDYASNQSSAAASSLRAGTDIDCGQTYPWHLNESFVAGEVSRGEIERSVTRLYANLVR
LGYFDKKNQYRSLGWKDVVKTDAWNISYEAAVEGIVLLKNDGTLPLSKKVRSIALIGPWANATT
QMQGNYYGPAPYLISPLEAAKKAGYHVNFELGTEIAGNSTTGFAKAIAAAKKSDAIIYLGGIDNTI
EQEGADRTDIAWPGNQLDLIKQLSEVGKPLVVLQMGGGQVDSSSLKSNKKVNSLVWGGYPG
QSGGVALFDILSGKRAPAGRLVTTQYPAEYVHQFPQNDMNLRPDGKSNPGQTYIWYTGKPVY

EFGSGLFYTTFKETLASHPKSLKFNTSSILSAPHPGYTYSEQIPVFTFEANIKNSGKTESPYTAML
FVRTSNAGPAPYPNKWLVGFDRLADIKPGHSSKLSIPIPVSALARVDSHGNRIVYPGKYELALNT
DESVKLEFELVGEEVTIENWPLEEQQIKDATPDA

Secreted protein broths expressing ABF1, ABF2, ABF3, AGL1, AGL2, AGL3, AXE1, AXE3, EG6, EG8, GLR1, MAN1, PEC2, XYN1, XYN3, and BXL1 were tested in ternary mixes. 150 µl AFEX-pretreated corn stover (31.7% glucan, 19.1 % xylan, 1.83% galactan, and 3.4% of arabinan, based on dry weight, made as either a 15.6 or 12% solids slurry in pH 5 50 mM sodium acetate buffer) was added to each well of a 96-well microtiter plate (all data points are based on triplicate wells). One experiment (shown in Table 9) employed dilute ammonia-pretreated corn cob at 13.84% solids as the substrate. To selected wells was added ACCELLERASE 1000^{™} (CEL) alone at 20 mg/G cellulose, ACCELLERASE 1000^{™} at 20 mg/G + 5 mg/G MULTIFECT^{®} Xylanase, or ACCELLERASE 1000^{™} at 20 mg/G + 5 mg/G MULTIFECT^{®} Xylanase + 1 or 5 mg/G of individual hemicellulase broths all in 20 µl total enzyme volume.

Enzyme doses were adjusted for total cellulose in either substrate slurry (15.6% or 12% solids). Plates were sealed and incubated with shaking at 50°C for 72 hours. Reactions were then quenched with 100 µl 100 mM glycine, pH 10. This mix was filtered and diluted an additional 6x (20 µl + 100 µl distilled H₂0) and analyzed for sugar content on an HPLC-Aminex HPX-87P column on an Agilent Chem Station HPLC instrument. HPLC peak areas were converted to sugar concentrations based on a cellobiose standard curve for cellobiose and glucose or on a xylose standard curve for xylose. Percent conversion based on starting cellulose content was calculated to include H₂O of hydrolysis for each of the three sugar polymers. Standard deviations of triplicates were also calculated.

Table 2 and 3 provide the mean conversion (± standard deviation) of glucans and of xylans for each enzyme mixture as determined in two separate executions of the protocol. These separate runs were performed with the two different AFEX substrate slurries of 15.6% (Table2) and 12 % solids (Table3) and thus include different total mgs of cellulose, though the dose as mg/G cellulose is the same.

**TABLE 2**

| **Enzyme** | | **%conversion Glucan (± SD)** | **%conversion xylan (± SD)** |
|---|---|---|---|
| 20 mp/G CEL | | 56.31 (0.88) | 39.47 (0.66) |
| 25 mp/G CEL | | 61.12 (0.99) | 41.82 (1.6) |
| 30 mp/G CEL | | 66.48 (1.9) | 46.69 (0.98) |
| 20 mg/G CEL + 5 mg/G XYN2 | - | 67.92 (1.1) | 61.02 (1.3) |
| | + 5 mg/G ABF1 | 68.84 (2.1) | 62.31 (0.67) |
| | + 5 mg/G ABF2 | 74.84 (2.4) | 62.36 (1.2) |
| | + 5 mg/G ABF3 | 72.96 (1.4) | 63.35 (3.7) |
| | + 5 mg/G AXE1 | 71.93 (1.4) | 64.78 (0.83) |
| | + 5 mg/G BXL1 | 78.45 (2.8) | 79.29 (4.9) |
| | + 5 mg/G EG6 | 70.15 (2.1) | 58.82 (2.7) |
| | + 5 mg/G GLR1 | 67.81 (1.8) | 65.70 (2.9) |
| | + 5 mg/G MAN1 | 74.58 (0.80) | 66.84 (0.64) |
| | + 5 mg/G PEC2 | 72.94 (4.3) | 61.99 (5.5) |
| | + 5 mg/G XYN1 | 67.33(1.1) | 62.22 (0.44) |
| | + 5 mg/G XYN3 | 78.82 (0.64) | 73.63 (0.50) |
| | + 1 mg/G XYN3 + 1 mg/G BXL1 | 77.37 (2.6) | 74.44 (2.3) |

**TABLE 3**

| **Enzyme** | | **%conversion glucan (± SD)** | **%conversion xylan (±SD)** |
|---|---|---|---|
| 20 mg/G CEL | | 55.08 (1.8) | 35.95 (0.94) |
| 30 mg/G CEL | | 62.63 (0.96) | 40.99 (0.30) |
| 20 mg/G CEL + 5 mg/G XYN2 | --- | **63.96 (0.58)** | **55.06 (2.0)** |
| | + 5 mg/G AGL1 | 67.52 (1.7) | 56.00 (1.2) |
| | + 5 mg/G AGL2 | 69.80 (2.7) | 55.02 (1.8) |
| | + 5 mg/G AGL3 | 66.51 (0.12) | 55.93 (0.59) |
| | + 5 mg/G AXE3 | 68.32 (1.4) | 55.89 (0.67) |
| | + 5 mg/G EG8 | 70.68 (3.9) | 55.40 (2.7) |

The addition of XYN2 was effective in increasing xylan conversion. Six enzyme mixtures with a third component (*i.e.*, XYN3, AGL2, EG8, BXL1, ABF3, or PEC2) showed further advantages in terms of glucan and/or xylan conversion compared to cellulase with XYN2. A quaternary enzyme mix was run according to the procedure described above. Table 4 provides the mean conversion (± standard deviation) of glucans and xylans for each enzyme mixture.

**TABLE 4**

| **Enzyme** | | **%conversion glucan (±SD)** | **%conversion xylan (±SD)** |
|---|---|---|---|
| 20 mg/G CEL | | 55.43 (6.5) | 42.29 (2.3) |
| 20 mg/G CEL + 5 mg/G XYN2 | | 71.27 (0.67) | 63.96 (1.2) |
| 20 mg/G CEL + 5 mg/G XYN3 | | 85.07 (3.1) | 68.69 (2.6) |
| 20 mg/G CEL + 5 XYN2 + 5 mg/G XYN3 | | 86.82 (1.2) | 80.68 (0.33) |

| **Enzyme** | | **%conversion glucan (±SD)** | **%conversion xylan (±SD)** |
|---|---|---|---|
| 20 mg/G CEL + 5 mg/G XYN2 + 2.5 XYN3 | --- | **76.57 (0.94)** | **72.70 (0.64)** |
| | + 5 mg/G ABF3 | 81.58 (0.76) | 75.89 (0.73) |
| | + 5 mg/G AGL2 | 78.66 (2.7) | 72.49 (2.3) |
| | + 5 mg/G BXL1 | 72.80 (6.7) | 78.60 (2.1) |
| | + 5 mg/G EG8 | 74.72 (6.0) | 73.29 (2.8) |
| | + 5 mg/G PEC2 | 78.18 (2.4) | 73.90 (2.9) |

In another experiment, ACCELLERASE 1000^{™} was mixed with purified XYN2 and/or XYN3 and assayed (Table 5). The combination of XYN2 and XYN3 produced more efficient glucan and xylan conversion.

**TABLE 5**

| **Enzyme** | | **%conversion glucan (±SD)** | **%conversion xylan (±SD)** |
|---|---|---|---|
| 10 mg/G CEL | --- | 43.97 (1.4) | 28.41 (1.0) |
| | + 10 mg/G XYN2 | 59.22 (4.3) | 56.83 (5.7) |
| | + 10 mg/G XYN3 | 51.44 (8.6) | 43.53 (1.6) |
| 20 mg/G CEL | --- | 60.29 (1.7) | 40.02 (0.33) |
| | + 5 mg/G XYN2 | 73.73 (0.79) | 61.81 (1.2) |
| | + 5 mg/G XYN3 | | |
| | + 10 mg/G XYN2 | 74.71 (1.6) | 65.20 (1.4) |
| | + 10 mg/G XYN3 | | |
| 30 mg/G CEL | | 67.05 (0.74) | 43.74 (0.14) |

In a further example, XYN4, XYN5, FAE1 and a new lot of ABF3 with ∼ 50% protein of interest (compared to previous lot at <10%) were tested as above in mixtures containing 20 mg/G ACCELLERASE 1000^{™} + 5 mg/G MULTIFECT^{®} Xylanase XYN2. The results are shown in Table 6. The addition of XYN4, XYN5, or FAE1 was effective in increasing the conversion of glucan and xylan.

**TABLE 6**

| **Enzyme** | | **%conversion glucan (± SD)** | **%conversion xylan (±SD)** |
|---|---|---|---|
| 20 mg/G CEL | | 57.52 (1.08) | 38.37 (0.38) |
| 30 mg/G CEL | | 66.21 (1.37) | 44.15 (0.70) |
| 20 mg/G CEL + 5 mg/G XYN2 | --- | **68.44 (0.23)** | **60.46 (0.48)** |
| | + 5 mg/G ABF3 | 66.22 (5.99) | 67.46 (3.97) |
| | + 5 mg/G XYN4 | 72.17 (0.66) | 63.47 (0.44) |
| | + 5 mg/G XYN5 | 71.91 (3.74) | 62.73 (3.37) |
| | + 5 mg/G FAE1 | 70.98 (1.47) | 67.02 (1.59) |

In another experiment, ACCELLERASE 1000^{™} was mixed with purified Bxl1 and XYN2 and/or XYN3 and assayed as above. The results are shown in Table 7. Several enzyme combinations were effective in increasing the conversion of glucan and/or xylan.

**TABLE 7**

| **Enzyme** | **%conversion glucan (± SD)** | **%conversion xylan (± SD)** |
|---|---|---|
| 35 mg/G CEL | 67.95 (0.67) | 40.36 (0.36) |
| 30 mg/G CEL | 66.51 (1.99) | 38.63 (0.56) |
| 20 mg/G CEL | 58.03 (3.19) | 32.28 (1.41) |
| 10 mg/g CEL | 45.01 (0.59) | 23.85 (0.42) |
| 10 mg/g CEL + 10 mg/G BXL1 | 46.89 (4.16) | 48.85 (2.94) |
| 20 CEL + 5 XYN2 + 5 BXL1 | 69.45 (4.88) | 60.15 (1.17) |
| 20 CEL + 5 XYN3 + 5 BXL1 | 65.17 (8.37) | 65.36 (1.14) |
| 20 CEL + 5 XYN2 + 5 XYN3 +5 BXL1 | 75.13 (1.20) | 66.97 (1.07) |

In another example, ABF1, ABF2 and ABF3 (ABF3 sample lot with <10% protein of interest), singly, in binary and ternary combinations were added to a background of 20 mg/G ACCELLERASE 1000^{™} + 5 mg/G purified XYN3 + 5 mg/G purified BXL1. The results are shown in Table 8. Several enzyme combinations were effective in increasing the conversion of glucan and/or xylan.

**TABLE 8**

| **Enzyme** | | **%conversion glucan (± SD)** | **%conversion xylan (±SD)** |
|---|---|---|---|
| 30 mg/G CEL | | 67.55 (0.18) | 45.05 (6.67) |
| 45 mg/G CEL | | 79.39 (4.66) | 56.05 (2.31) |
| | --- | **73.24 (4.39)** | **79.88 (4.72)** |
| 20 mg/G CEL | + 5 mg/G ABF1 | 58.21 (0.55) | 86.84 (0.47) |
| + 5 mg/GXYN3 | + 5 mg/G ABF2 | 84.39 (1.01) | 87.15 (1.32) |
| + 5 mg/G BXL1 | + 5 mg/G ABF3 | 65.07 (3.68) | 73.46 (4.13) |
| | + 5 mg/GABF1 | 87.65 (3.11) | 87.08 (2.31) |
| | +5 mg/G ABF2 | | |
| | + 5 mg/GABF1 | 67.62 (5.01) | 87.77 (2.91) |
| | +5 mg/G ABF3 | | |
| | + 5 mg/GABF2 | 91.21 (1.82) | 89.98 (1.08) |
| | +5 mg/G ABF3 | | |
| | + 5 mg/GABF1 | 99.67 (3.45) | 96.73 (4.74) |
| | +5 mg/G ABF2 | | |
| | + 5 mg/G ABF3 | | |

In another example 3.4 mg/G xylan of purified ABF1, ABF2 and/or ABF3 were added to a 20.7 mg/G glucan of ACCELLERASE 1000^{™} + 5.1 mg/G xylan each of purified XYN3 and BXL1. The results are shown in Table 9. Several enzyme combinations were effective in increasing the conversion of glucan and/or xylan.

**TABLE 9**

| **Enzyme** | | **%conversion glucan (± SD)** | **%conversion xylan (±SD)** |
|---|---|---|---|
| 30.9 mg/G CEL | | 66.45 (1.64) | 33.84 (0.83) |
| 41.3 mg/G CEL | | 67.99 (0.57) | 35.95 (0.11) |
| | --- | **76.67 (0.30)** | **63.86 (0.08)** |
| | +3.4 mg/G ABF1 | 76.37 (1.32) | 64.18 (1.77) |
| | +3.4 mg/G ABF2 | 77.84 (1.48) | 66.59 (2.07) |
| | +3.4 mg/G ABF3 | 77.53 (1.94) | 66.86 (1.84) |
| | +3.4 mg/G ABF1 | 78.32 (1.56) | 67.65 (2.31) |
| 20.7 mg/G CEL | +3.4 mg/G ABF2 | | |
| + 5.1 mg/GXYN3 | + 3.4 mg/G ABF1 | 77.53 (1.04) | 66.89 (0.51) |
| + 5.1 mg/G BXL1 | +3.4 mg/G ABF3 | | |
| | +3.4 mg/G ABF2 | 79.92 (0.27) | 68.96 (0.03) |
| | +3.4 mg/G ABF3 | | |
| | + 3.4 mg/G ABF1 | 80.22 (1.98) | 68.76 (222) |
| | +3.4 mg/G ABF2 | | |
| | + 3.4 mg/G ABF3 | | |

## Claims

1. An enzyme blend composition for hydrolyzing a mixture of cellulosic and hemicellulosic materials, comprising:
(a) a first enzyme composition comprising a whole cellulase,
(b) a second enzyme composition comprising at least one xylanase selected from a GH10 or GH11 xylanase, and
(c) a third enzyme composition comprising at least one additional hemicellulase,
wherein the at least one additional hemicellulase is a combination of a β-xytosidase and an arabinofuranosidase,
wherein the β-xylosidase is BXL1 from *Trichoderma reesei* and the arabinofuranosidase is α-arabinofuranosidase III (ABF3) from *Trichoderma reesei,*
wherein the amount of hemicellulase is in the range of 1 % to 50% of the total protein and the whole cellulase is in the range of less than 99% to 50% of total protein, and.
wherein the enzyme blend composition provides at least one of (i) enhanced glucan conversion or (ii) enhanced xylan conversion compared to an equivalent enzyme blend composition lacking the at least one additional hemicellulase.

2. The composition of claim 1, wherein the first enzyme composition is a whole cellulase blend from a filamentous fungus.

3. The composition of claim 1, wherein the first enzyme composition is a whole cellulase blend from a filamentous fungus supplemented with an addition amount of β-glucosidase.

4. The composition of any of the preceding claims, wherein the second enzyme composition comprises xylanase XYN2 from *Trichoderma reesei.*

5. The composition of any of the preceding claims, wherein the second enzyme composition comprises xylanase XYN3 from *Trichoderma reesei.*

6. The composition of any of the preceding claims, wherein the at least one xylanase has an amino acid sequence having at least 80% identity to an amino acid sequence selected from SEQ ID NO: 1 or SEQ ID NO: 2.

7. The composition of any of the preceding claims, wherein the first enzyme composition is a whole cellulase blend from a filamentous fungus supplemented with an addition amount of β-glucosidase, the second enzyme composition comprises a xylanase, and the at least one additional hemicellulase is a combination of a β-xylosidase and arabinofuranosidase.

8. The composition of any of claims 1 to 7, wherein the at least one additional hemicellulose comprises α-arabinofuranosidase I (ABF1), α-arabinofuranosidase II (ABF2) and ABF3.

9. The composition of any of the preceding claims, wherein the at least one additional hemicellulase has an amino acid sequence having at least 80% identity to an amino acid sequence selected from the group consisting of SEQ ID NO: 3, SEQ ID NO: 4, SEQ ID NO: 5, and SEQ ID NO: 17.

10. A method for hydrolyzing a mixture of cellulosic and hemicellulosic materials, comprising contacting the mixture of cellulosic and hemicellulosic materials with:
(a) a first enzyme composition comprising a whole cellulase,
(b) a second enzyme composition comprising at least one xylanase selected from a GH10 or GH11 xylanase, and
(c) a third enzyme composition comprising at least one additional hemicellulase
wherein the at least one additional hemicellulase is a combination of a β-xylosidase and an arabinofuranosidase,
wherein the β-xylosidase is BXL1 from *Trichoderma reesei* and the arabinofuranosidase is α-arabinofuranosidase III (ABF3) from *Trichoderma reesei,*
wherein the amount of hemicellulase is in the range of 1 % to 50% of the total protein and the whole cellulase is in the range of less than 99% to 50% of total protein,
thereby hydrolyzing the mixture of cellulosic and hemicellulosic materials,
wherein the contacting results in at least one of (i) enhanced glucan conversion or (ii) enhanced xylan conversion compared to equivalent contacting in the absence of the at least one additional hemicellulase.

11. The method of claim 10, wherein the first enzyme composition, the second enzyme composition and/or the third enzyme composition are as defined in any one of claims 2 to 9.

12. The method of claim 10 or claim 11, wherein contacting the mixture of cellulosic and hemicellulosic materials with the first enzyme composition, the second enzyme composition, and the third enzyme composition are performed simultaneously.

13. The method of claim 12, wherein the first enzyme composition, the second enzyme composition, and the third enzyme composition are provided in a single composition enzyme blend.

## Patentansprüche

1. Enzymblendzusammensetzung zum Hydrolysieren eines Gemischs aus cellulosischen und hemicellulosischen Materialien, umfassend:
(a) eine erste Enzymzusammensetzung, die eine Vollcellulase umfasst,
(b) eine zweite Enzymzusammensetzung, die zumindest eine Xylanase, ausgewählt aus einer GH10- oder einer GH11-Xylanase, umfasst, und
(c) eine dritte Enzymzusammensetzung, die zumindest eine zusätzliche Hemicellulase umfasst,
wobei die zumindest eine zusätzliche Hemicellulase eine Kombination aus einer β-Xylosidase und einer Arabinofuranosidase ist,
wobei die β-Xylosidase BXL1 von Trichoderma reesei ist und die Arabinofuranosidase α-Arabinofuranosidase III (ABF3) von Trichoderma reesei ist,
wobei die Hemicellulasemenge im Bereich von 1 % bis 50 % des gesamten Proteins liegt und die Vollcellulase im Bereich von weniger als 99 % bis 50 % des gesamten Proteins vorliegt und
wobei die Enzymblendzusammensetzung zumindest eines von (i) verbesserter Glucanumwandlung oder (ii) verbesserter Xylanumwandlung im Vergleich zu einer äquivalenten Enzymblendzusammensetzung liefert, der die zumindest eine zusätzliche Hemicellulase fehlt.

2. Zusammensetzung nach Anspruch 1, wobei die erste Enzymzusammensetzung ein Vollcellulaseblend von einem Fadenpilz ist.

3. Zusammensetzung nach Anspruch 1, wobei die erste Enzymzusammensetzung ein Vollcellulaseblend von einem Fadenpilz, ergänzt mit einer Zusatzmenge β-Glucosidase ist.

4. Zusammensetzung nach einem der vorangegangenen Ansprüche, wobei die zweite Enzymzusammensetzung Xylanase XYN2 von Trichoderma reesei umfasst.

5. Zusammensetzung nach einem der vorangegangenen Ansprüche, wobei die zweite Enzymzusammensetzung Xylanase XYN3 von Trichoderma reesei umfasst.

6. Zusammensetzung nach einem der vorangegangenen Ansprüche, wobei die zumindest eine Xylanase eine Aminosäuresequenz aufweist, die mindestens 80 % Identität mit einer Aminosäuresequenz, ausgewählt aus SEQ ID NO: 1 oder SEQ ID NO: 2, besitzt.

7. Zusammensetzung nach einem der vorangegangenen Ansprüche, wobei die erste Enzymzusammensetzung ein Vollcellulaseblend von einem Fadenpilz ist, ergänzt mit einer Zusatzmenge β-Glucosidase, die zweite Enzymzusammensetzung eine Xylanase umfasst und die zumindest eine zusätzliche Hemicellulase eine Kombination aus einer β-Xylosidase und einer Arabinofuranosidase ist.

8. Zusammensetzung nach einem der Ansprüche 1 bis 7, wobei die zumindest eine zusätzliche Hemicellulase α-Arabinofuranosidase I (ABF1), α-Arabinofuranosidase II (ABF2) und ABF3 umfasst.

9. Zusammensetzung nach einem der vorangegangenen Ansprüche, wobei die zumindest eine zusätzliche Hemicellulase eine Aminosäuresequenz aufweist, die mindestens 80 % Identität mit einer Aminosäuresequenz besitzt, die aus der Gruppe, bestehend aus SEQ ID NO: 3, SEQ ID NO: 4, SEQ ID NO: 5 und SEQ ID NO: 17, ausgewählt ist.

10. Verfahren zum Hydrolysieren eines Gemischs aus cellulosischen und hemicellulosischen Materialien, umfassend In-Kontakt-Bringen des Gemischs aus cellulosischen und hemicellulosischen Materialien mit:
(a) einer ersten Enzymzusammensetzung, die eine Vollcellulase umfasst,
(b) einer zweiten Enzymzusammensetzung, die zumindest eine Xylanase, ausgewählt aus einer GH10- oder einer GH11-Xylanase, umfasst, und
(c) einer dritten Enzymzusammensetzung, die zumindest eine zusätzliche Hemicellulase umfasst,
wobei die zumindest eine zusätzliche Hemicellulase eine Kombination aus einer β-Xylosidase und einer Arabinofuranosidase ist,
wobei die β-Xylosidase BXL1 von Trichoderma reesei ist und die Arabinofuranosidase α-Arabinofuranosidase III (ABF3) von Trichoderma reesei ist,
wobei die Hemicellulasemenge im Bereich von 1 % bis 50 % des gesamten Proteins liegt und die Vollcellulase im Bereich von weniger als 99 % bis 50 % des gesamten Proteins vorliegt,
dadurch Hydrolysieren des Gemischs aus cellulosischen und hemicellulosischen Materialien,
wobei das In-Kontakt-Bringen zumindest eines von (i) verbesserter Glucanumwandlung oder (ii) verbesserter Xylanumwandlung im Vergleich zu äquivalentem In-Kontakt-Bringen bei Nicht-Vorhandensein der zumindest einen zusätzlichen Hemicellulase ergibt.

11. Verfahren nach Anspruch 10, wobei die erste Enzymzusammensetzung, die zweite Enzymzusammensetzung und/oder die dritte Enzymzusammensetzung wie in einem der Ansprüche 2 bis 9 definiert sind.

12. Verfahren nach Anspruch 10 oder Anspruch 11, wobei das In-Kontakt-Bringen des Gemischs aus cellulosischen und hemicellulosischen Materialien mit der ersten Enzymzusammensetzung, der zweiten Enzymzusammensetzung und der dritten Enzymzusammensetzung simultan durchgeführt wird.

13. Verfahren nach Anspruch 12, wobei die erste Enzymzusammensetzung, die zweite Enzymzusammensetzung und die dritte Enzymzusammensetzung in einem Enzymblend einer einzigen Zusammensetzung bereitgestellt werden.

## Revendications

1. Composition de mélange d'enzymes pour l'hydrolyse d'un mélange de matériaux cellulosiques et hémicellulosiques, comprenant :
(a) une première composition d'enzyme comprenant une cellulase entière,
(b) une deuxième composition d'enzyme comprenant au moins une xylanase choisie parmi une xylanase GH10 ou GH11, et
(c) une troisième composition d'enzyme comprenant au moins une hémicellulase supplémentaire,
dans laquelle la au moins une hémicellulase supplémentaire est une combinaison d'une β-xylosidase et d'une arabinofuranosidase,
dans laquelle la β-xylosidase est BXL1 provenant de *Trichoderma reesei* et l'arabinofuranosidase est l'α-arabinofuranosidase III (ABF3) provenant de *Trichoderma reesei,*
dans laquelle la quantité d'hémicellulase est dans l'intervalle de 1 % à 50 % de la protéine totale et la cellulase entière est dans l'intervalle de moins de 99 % à 50 % de la protéine totale, et
dans laquelle la composition de mélange d'enzymes fournit au moins une parmi (i) une conversion de glucane améliorée ou (ii) une conversion de xylane améliorée comparée à une composition de mélange d'enzymes équivalente dénuée de la au moins une hémicellulase supplémentaire.

2. Composition selon la revendication 1, dans laquelle la première composition d'enzyme est un mélange de cellulases entières provenant d'un champignon filamenteux.

3. Composition selon la revendication 1, dans laquelle la première composition d'enzyme est un mélange de cellulases entières provenant d'un champignon filamenteux complétée avec une quantité supplémentaire de β-glucosidase.

4. Composition selon l'une quelconque des revendications précédentes, dans laquelle la deuxième composition d'enzyme comprend la xylanase XYN2 provenant de *Trichoderma reesei.*

5. Composition selon l'une quelconque des revendications précédentes, dans laquelle la deuxième composition d'enzyme comprend la xylanase XYN3 provenant de *Trichoderma reesei.*

6. Composition selon l'une quelconque des revendications précédentes, dans laquelle la au moins une xylanase possède une séquence d'acides aminés ayant au moins 80 % d'identité avec une séquence d'acides aminés choisie parmi SEQ ID NO : 1 ou SEQ ID NO : 2.

7. Composition selon l'une quelconque des revendications précédentes, dans laquelle la première composition d'enzyme est un mélange de cellulases entières provenant d'un champignon filamenteux complétée avec une quantité d'ajout de β-glucosidase, la deuxième composition d'enzyme comprend une xylanase, et la au moins une hémicellulase supplémentaire est une combinaison d'une β-xylosidase et d'une arabinofuranosidase.

8. Composition selon l'une quelconque des revendications 1 à 7, dans laquelle au moins une hémicellulose supplémentaire comprend de l'a-arabinofuranosidase I (ABF1), de l'a-arabinofuranosidase II (ABF2) et de l'ABF3.

9. Composition selon l'une quelconque des revendications précédentes, dans laquelle la au moins une hémicellulase supplémentaire possède une séquence d'acides aminés ayant au moins 80 % d'identité avec une séquence d'acides aminés choisie dans le groupe constitué de SEQ ID NO : 3, SEQ ID NO : 4, SEQ ID NO : 5, et SEQ ID NO : 17.

10. Procédé pour l'hydrolyse d'un mélange de matériaux cellulosiques et hémicellulosiques, comprenant une mise en contact du mélange de matériaux cellulosiques et hémicellulosiques avec :
(a) une première composition d'enzyme comprenant une cellulase entière,
(b) une deuxième composition d'enzyme comprenant au moins une xylanase choisie parmi une xylanase GH10 ou GH11, et
(c) une troisième composition d'enzyme comprenant au moins une hémicellulase supplémentaire,
dans lequel la au moins une hémicellulase supplémentaire est une combinaison d'une β-xylosidase et d'une arabinofuranosidase,
dans lequel la β-xylosidase est BXL1 provenant de *Trichoderma reesei* et l'arabinofuranosidase est l'α-arabinofuranosidase III (ABF3) provenant de *Trichoderma reesei,*
dans lequel la quantité d'hémicellulase est dans l'intervalle de 1 % à 50 % de la protéine totale et la cellulase entière est dans l'intervalle de moins de 99 % à 50 % de la protéine totale,
en hydrolysant ainsi le mélange de matériaux cellulosiques et hémicellulosiques,
dans lequel la mise en contact conduit à au moins une parmi (i) une conversion de glucane améliorée ou (ii) une conversion de xylane améliorée comparée à une mise en contact équivalente en l'absence de la au moins une hémicellulase supplémentaire.

11. Procédé selon la revendication 10, dans lequel la première composition d'enzyme, la deuxième composition d'enzyme et/ou la troisième composition d'enzyme sont telles que définies dans l'une quelconque des revendications 2 à 9.

12. Procédé selon la revendication 10 ou la revendication 11, dans laquelle des mises en contact du mélange de matériaux cellulosiques et hémicellulosiques avec la première composition d'enzyme, la deuxième composition d'enzyme et la troisième composition d'enzyme sont effectuées simultanément.

13. Procédé selon la revendication 12, dans lequel la première composition d'enzyme, la deuxième composition d'enzyme et la troisième composition d'enzyme sont fournies dans un mélange d'enzymes à une seule composition.
